# EUROPEAN PATENT APPLICATION

(11) **EP 4 729 113 A1**
(43) Date of publication of application: **22.04.2026**
(21) Application number: 24857479.0
(22) Date of filing: 16.08.2024
(51) Int. Cl.: A61N 1/36

(54) **METHOD FOR CONTROLLING HIGH-FREQUENCY OUTPUT APPARATUS ON BASIS OF PRESSURE SENSOR, TREATMENT HEAD, AND HIGH-FREQUENCY OUTPUT APPARATUS**

(30) Priority: 15.08.2024 CN 202411123389
(71) Applicant: Shenzhen Peninsula Medical Group, Shenzhen, Guangdong 518000 (CN)
(72) Inventor: HU, Shuyun, Shenzhen, Guangdong 518000 (CN); QIN, Zhao, Shenzhen, Guangdong 518000 (CN); LIU, Xiaoxing, Shenzhen, Guangdong 518000 (CN)
(74) Representative: Osterhoff, Utz
(86) International application number: PCT/CN2024/112799
(87) International publication number: WO 2026/036392

(57) **Abstract**

Disclosed are a method for controlling a high-frequency output device based on a pressure-sensitive sensor, a treatment tip, and a high-frequency output device, relating to the technical field of high-frequency output devices. The pressure-sensitive sensor is provided at the treatment tip of the high-frequency output device, and the treatment tip is configured to contact skin. The method includes: step S10, obtaining a contact signal collected by the pressure-sensitive sensor, the contact signal being generated while the treatment tip contacts the skin; and step S20, determining a contact condition between the treatment tip and the skin according to the contact signal, controlling the high-frequency output device to output an operation power and/or an operation frequency with a corresponding value to the treatment tip.

## Description

### CROSS-REFERENCE TO RELATED APPLICATIONS

The present application claims priority to Chinese Patent Application No. 202411123389.9, filed on August 15, 2024, and titled "METHOD FOR CONTROLLING HIGH-FREQUENCY OUTPUT DEVICE BASED ON PRESSURE-SENSITIVE SENSOR, TREATMENT TIP AND HIGH-FREQUENCY OUTPUT DEVICE". The disclosures of the above-mentioned applications are incorporated herein by reference in their entireties.

### TECHNICAL FIELD

The present application relates to the technical field of high-frequency output devices, and in particular to a method for controlling a high-frequency output device based on a pressure-sensitive sensor, a treatment tip, and a high-frequency output device.

### BACKGROUND

At present, the treatment tip of the high-frequency output device usually uses high-frequency energy for treatment. The high-frequency energy treatment technology is a method that uses energy such as high-frequency ultrasound and high-frequency alternating current for achieving non-invasive treatment. This technology transmits high-frequency energy waves to a specific small area below the epidermis to produce local thermal effects or other physical effects, thereby achieving the treatment purpose. It should be understood that the conventional treatment tip cannot perform treatment at the appropriate time, resulting in poor clinical and treatment effects in some application scenarios. For example, the treatment tip cannot perform the corresponding treatment in a specific situation, or cannot output the corresponding operation frequency or operation power in a specific situation, which leads to accidental injury to the skin and inability to perform efficient treatment.

The existing solution is to add multiple sensors to the treatment tip to detect the different movements of the treatment tip on the skin, so as to perform the treatment at the appropriate time. However, it should be noted that the internal space of the treatment tip is provided with multiple sensors, which will lead to a high internal space occupancy rate, thereby reducing the internal space of the treatment tip and increasing the cost. In addition, misjudgment and low sensitivity will also occur in the treatment tip.

### SUMMARY

The main purpose of the present application is to propose a method for controlling a high-frequency output device based on a pressure-sensitive sensor, a treatment tip and a high-frequency output device, aiming to reduce the internal space occupied by the treatment tip while improving the treatment effect of the treatment tip.

In view of the above objectives, the present application provides a method for controlling a high-frequency output device based on a pressure-sensitive sensor. The high-frequency output device includes a treatment tip configured to contact skin and transmit high-frequency energy to the skin, the pressure-sensitive sensor is provided at the treatment tip, and the method includes:
step S10, obtaining a contact signal collected by the pressure-sensitive sensor, the contact signal being generated when the treatment tip contacts the skin; and
step S20, determining a contact condition between the treatment tip and the skin according to the contact signal, to control the high-frequency output device to output an operation power and/or an operation frequency with a corresponding value to the treatment tip.

In an embodiment, the contact signal includes a first signal and a second signal, the first signal represents sliding speed information of the treatment tip contacting with the skin, and the second signal represents pressing force information of the treatment tip contacting with the skin.

In an embodiment, step S20 further includes:
in response to that a value corresponding to the first signal is less than a first preset threshold which indicates that the treatment tip is in static contact with the skin, outputting a first parameter to the treatment tip by the high-frequency output device;
in response to that a value corresponding to the first signal is greater than a first preset threshold which indicates that the treatment tip is in dynamic contact with the skin, outputting a second parameter to the treatment tip by the high-frequency output device.

In an embodiment, step S20 further includes:
in response to that the value corresponding to the first signal is greater than the first preset threshold and less than a second preset threshold which indicates that the treatment tip is in contact with the skin at a first sliding speed, outputting a third parameter to the treatment tip by the high-frequency output device;
in response to that the value corresponding to the first signal is greater than a second preset threshold which indicates that the treatment tip is in contact with the skin at a second sliding speed, outputting a fourth parameter to the treatment tip by the high-frequency output device.

In an embodiment, step S20 further includes: in response to that the value corresponding to the first signal is greater than the first preset threshold, and the value corresponding to the first signal changes continuously, an output power of the high-frequency output device changes continuously along with the change of the value corresponding to the first signal.

In an embodiment, step S20 further includes:
in response to that a value corresponding to the second signal is less than a third preset threshold which indicates that the treatment tip does not effectively contact with the skin, outputting a fifth parameter to the treatment tip by the high-frequency output device;
in response to that a value corresponding to the second signal is greater than a third preset threshold which indicates that the treatment tip is in static contact with the skin, outputting a first parameter to the treatment tip by the high-frequency output device.

In an embodiment, step S20 further includes:
step S21, determining whether a value corresponding to the second signal is less than a third preset threshold, if the value corresponding to the second signal is less than the third preset threshold which indicates that the treatment tip does not effectively contact with the skin, outputting a fifth parameter to the treatment tip by the high-frequency output device, if the value corresponding to the second signal is not less than the third preset threshold, executing a next step; and
step S22, determining whether a value corresponding to the first signal is less than a first preset threshold, if the value corresponding to the first signal is less than the first preset threshold which indicates that the treatment tip is in static contact with the skin, outputting a first parameter to the treatment tip by the high-frequency output device, if the value corresponding to the first signal is not less than the first preset threshold which indicates that the treatment tip is in dynamic contact with the skin, and outputting a second parameter to the treatment tip by the high-frequency output device.

In an embodiment, after step S22, the method further includes:
step S23, in response to that a value corresponding to the second signal is greater than the third preset threshold, and the value corresponding to the first signal is greater than the first preset threshold, determining whether the value corresponding to the first signal is less than the second preset threshold, if the value corresponding to the first signal is less than the second preset threshold which indicates that the treatment tip is in contact with the skin at a first sliding speed, outputting a third parameter to the treatment tip by the high-frequency output device, if the value corresponding to the first signal is not less than the second preset threshold which indicates that the treatment tip is in contact with the skin at a second sliding speed, outputting a fourth parameter to the treatment tip by the high-frequency output device.

The second preset threshold is greater than the first preset threshold, and the second sliding speed is greater than the first sliding speed.

In an embodiment, the first signal indicates a change rate of an operation voltage and/or an operation current; and the second signal indicates an amplitude of the operation voltage and/or the operation current.

The present application further provides a treatment tip. In an embodiment, the treatment tip includes:
a pressure-sensitive sensor;
a memory for storing a computer program; and
a processor for executing the computer program.

The computer program is configured to implement the method for controlling a high-frequency output device based on a pressure-sensitive sensor according to any embodiment as mentioned above.

The present application further provides a high-frequency output device. In an embodiment, the high-frequency output device includes: an output module; a feedback module; a control module; and the treatment tip as mentioned above. The feedback module is configured to receive and transmit a contact signal outputted by the pressure-sensitive sensor of the treatment tip, the control module is electrically connected to the feedback module and the treatment tip, and the control module is configured to receive a contact signal to control the output module to output an operation power and/or an operation frequency with a corresponding value to the treatment tip.

The present application proposes a method for controlling a high-frequency output device based on a pressure-sensitive sensor. The pressure-sensitive sensor is used in the treatment tip of a high-frequency output device. When the treatment tip contacts the skin, the pressure-sensitive sensor can generate a corresponding contact signal according to the contact mode between the treatment tip and the skin. Thus, the contact mode between the treatment tip and the skin can be determined by the contact signal (the contact signal includes: a value corresponding to the first signal and a value corresponding to the second signal, and the value corresponding to the first signal represents the sliding speed information of the treatment tip contacting the skin; the value corresponding to the second signal represents the pressing force information of the treatment tip contacting the skin), and the control module of the high-frequency output device can control the operation parameters of the treatment tip according to the contact mode. Compared with the arts, the treatment is performed once the treatment tip contacts the skin, and corresponding working parameters cannot match the user's sliding speed during use (operation power and/or operation frequency), resulting in poor treatment. The present application provides the method for controlling multiple parameters detected using the pressure-sensitive sensor. The corresponding treatment mode is selected to perform based on the contact status between the treatment tip and the skin, and the corresponding treatment parameters are also determined no matter how quickly or slowly the treatment tip slides on the skin, to ensure that the sliding speeds match the treatment parameters, also making sure that the treatment tip stops working when it is not needed once the treatment tip is in static contact, thereby improving the treatment effect of the high-frequency output device or the treatment tip. In addition, the present application uses only one pressure-sensitive sensor to realize the detection of the pressing force and the sliding speed of the treatment tip, reducing the installation space inside the treatment tip. In this way, compared with the treatment tip in the conventional technology, the present application reduces the installation space inside the treatment tip as well as improves the treatment effect of the treatment tip.

### BRIEF DESCRIPTION OF THE DRAWINGS

To more clearly illustrate technical solutions in the embodiments of the present application or the related art, the following will briefly introduce the drawings that need to be used in the description of the embodiments or the related art. Obviously, the drawings in the following description are only some embodiments of the present application. For those skilled in the art, without creative effort, other drawings can be obtained according to the structures shown in these drawings.
FIG. 1 is a flowchart of a method for controlling an operation parameter according to an embodiment of the present application.
FIG. 2 is a flowchart of the method for controlling an operation parameter according to another embodiment of the present application.

The realization of the objective, functional characteristics, and advantages of the present application are further described with reference to the accompanying drawings.

### DETAILED DESCRIPTION OF THE EMBODIMENTS

The technical solutions of the embodiments of the present application will be described in detail below with reference to the accompanying drawings. It is obvious that the embodiments described are only some rather than all of the embodiments of the present application. All other embodiments obtained by those skilled in the art based on the embodiments of the present application without creative efforts shall fall within the claimed scope of the present application.

It should be noted that all the directional indications (such as up, down, left, right, front, rear...) in the embodiments of the present application are only used to explain the relative positional relationship, movement, or the like of the components in a certain posture. If the specific posture changes, the directional indication will change accordingly.

Besides, the descriptions associated with, e.g., "first" and "second," in the present application are merely for descriptive purposes, and cannot be understood as indicating or suggesting relative importance or implicating the number of the indicated technical feature. Therefore, the feature associated with "first" or "second" can expressly or implicate include at least one such feature. In addition, the technical solutions of the various embodiments can be combined with each other, but the combinations must be based on the realization of those skilled in the art. When the combination of technical solutions is contradictory or cannot be achieved, it should be considered that such a combination of technical solutions does not exist, nor does it fall within the scope of the present application.

At present, the treatment tip of the high-frequency output device usually uses high-frequency energy for treatment. The high-frequency energy treatment technology is a method that uses high-frequency energy such as high-frequency ultrasound, high-frequency alternating current, and high-frequency electromagnetic waves for achieving non-invasive treatment. This technology transmits high-frequency energy waves to a specific small area below the epidermis to produce local thermal effects or other physical effects, thereby achieving the treatment purpose. It can be understood that the high-frequency output device mentioned in this application, including an ultrasonic treatment device, a high-frequency current treatment device (or a radio frequency treatment device), and the like, requires good contact between the treatment tip and the surface of the skin tissue. The conventional treatment tip cannot perform treatment at the appropriate time, resulting in poor clinical and treatment effects in some application scenarios. For example, the treatment tip cannot perform the corresponding treatment in a specific situation, or cannot output the corresponding operation frequency or operation power in a specific situation, which leads to accidental injury to the skin and inability to perform efficient treatment.

The existing solution is to add multiple sensors to the treatment tip to detect the different movements of the treatment tip on the skin, so as to perform treatment at the appropriate time. However, it should be noted that the internal space of the treatment tip is provided with multiple sensors, which will lead to a high internal space occupancy rate, thereby reducing the internal space of the treatment tip and increasing the cost. In addition, for the treatment tip, one sensor will be used for conventional attachment judgment, and another sensor will be used for movement judgment. In this way, if the movement is judged through a simple displacement sensor, it will also cause misjudgment when the user is walking, increasing the medical risk such as insensitivity and false triggering, and still cannot perform efficient treatment.

In addition, some problems still occur in the treatment tip of the related art. Specifically, the treatment tip fails to start or adjust its operation parameters (such as frequency and power) at the best time, resulting in poor treatment effect and even possible harm to the patient. For example, during the treatment process, if the treatment tip is activated when it is not fully attached to the skin or is stationary on the skin, it may cause local overheating, thereby causing skin burns or tissue damage, especially for sensitive areas such as areas around the eyes. In addition, if treatment is performed when the treatment tip is in poor contact with the skin, uneven energy distribution may lead to unsatisfactory treatment effects or even deviate from the target area, causing unnecessary medical risks.

Therefore, there is a need for a treatment tip that can reduce the internal space occupied by the treatment tip while improving the treatment effect of the treatment tip.

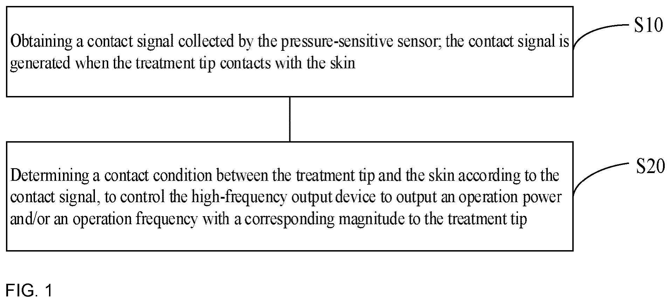

Therefore, in order to improve the treatment effect of the treatment tip while reducing the internal space occupied by the treatment tip, the present application proposes a method for controlling an operation parameter based on a pressure-sensitive sensor. In an embodiment, as shown in FIG. 1, the pressure-sensitive sensor is arranged on the treatment tip of a high-frequency output device, and the treatment tip is used to contact the skin. The method includes steps S10 and S20. Step S10, obtaining a contact signal collected by the pressure-sensitive sensor. The contact signal is generated when the treatment tip contacts the skin. Step S20, determining a contact condition between the treatment tip and the skin according to the contact signal, to control the high-frequency output device to output an operation power and/or an operation frequency with a corresponding value to the treatment tip. In this way, by monitoring the contact condition between the treatment tip and the skin (which usually refers to the patient's skin or lesion area), the treatment parameters of the high-frequency output device can be dynamically adjusted to ensure that the treatment is effective and safe.

It can be understood that the contact condition can be divided into multiple types, which can be no contact, static contact, and dynamic contact. Dynamic contact can be distinguished according to the sliding speed. Each contact state can correspond to an operation parameter, so that the treatment effect of the treatment tip can be improved.

The sensor includes but not limited to: a resistive sensor, a capacitive sensor, or other type of sensor.

For step S10, obtaining a contact signal collected by the pressure-sensitive sensor, the contact signal is generated once the treatment tip contacts the skin, and this step involves collecting data from the pressure-sensitive sensor installed at the treatment tip. When the treatment tip contacts the skin, the pressure-sensitive sensor generates an electrical signal according to the contact pressure and/or sliding conditions, and the electrical signal directly reflects the specific details of the contact between the treatment tip and the skin, such as the pressing force and the sliding speed. It is understandable that the original electrical signal generated by the pressure-sensitive sensor is usually an analog signal. These signals need to be converted into digital signals by an analog-to-digital converter (ADC) for subsequent computer processing.

For step S20, determining a contact condition between the treatment tip and the skin according to the contact signal, to control the high-frequency output device to output an operation power and/or an operation frequency with a corresponding value to the treatment tip. In this step, the control system of the high-frequency output device will analyze the contact signal obtained in step S10 to determine the contact mode between the treatment tip and the skin. The control system will analyze the contact signal to identify whether the treatment tip is in a stationary state or a sliding state, and to determine the value of the sliding speed and the pressing force. Based on the above information, the system will decide how to adjust the operation parameters (operation power and/or operation frequency) of the treatment tip. For example, if the treatment tip is sliding quickly, the intensity or frequency of the ultrasound may should be increased to ensure uniform treatment of the treatment tip and avoid poor treatment effect due to too fast movement. On the contrary, if the treatment tip is attached to the skin but keeps stationary on the skin, the outputted ultrasound energy can be reduced or turned off to avoid injuring the user.

In an actual application scenario, in order to improve the treatment effect and safety of the treatment, a pressure-sensitive sensor is integrated on the treatment tip. When the treatment tip contacts the skin, the resistance value of the pressure-sensitive sensor will change according to the contact condition, thereby the corresponding operation voltage or operation current will also change. The change of the operation voltage or the operation current is transmitted to the control system for analyzing, and the sliding speed and pressure of the treatment tip can be extracted. Then, the control system automatically adjusts the operation parameters of the treatment tip, such as the power and the frequency of the ultrasound, according to the information. Specifically, when the therapist makes the treatment tip attached to the skin and moves the treatment tip slowly, the resistance value of the pressure-sensitive sensor will first change suddenly and then change slowly, and the corresponding operation voltage will change accordingly. Based on the change in the operation voltage, the system will determine that the treatment tip is sliding slowly after the treatment tip is attached to the skin, and will reduce the output power of the ultrasound to avoid over-treatment based on this. On the contrary, when the treatment tip is attached to the target issue and moves quickly, the resistance value of the pressure-sensitive sensor will first change suddenly and then change slowly, and the corresponding operation voltage will change accordingly. The system will determine that the treatment tip is sliding quickly after it is attached to the skin based on the change in the operation voltage, and the system will increase the power of the ultrasound to ensure adequate treatment. In this way, based on the dynamic adjustment mechanism of the operation parameters of the pressure-sensitive sensor, the high-frequency output device can use energy more efficiently, reduce side effects, improve treatment efficiency and safety, and also improve the patient's treatment experience.

It should be noted that the treatment tip in the related art uses a variety of sensors, such as proximity sensors, accelerometers, gyroscopes, temperature sensors, pressure sensors, and the like. These sensors are used for detecting different physical properties, such as distance, motion state, rotation angle, local temperature, and pressure distribution. However, this arrangement with multi sensors not only increases the structural complexity and cost of the treatment tip, but also occupies internal space, limiting the integration and optimization of other key components. To this end, in this embodiment, only a pressure-sensitive sensor is used to simultaneously sense the contact pressure and the sliding speed, thereby simplifying the sensor configuration, reducing the space that sensor occupies in the treatment tip, and improving the treatment effect.

In this embodiment, there can be multiple pressure-sensitive sensors. Specifically, multiple sensors are arranged at the end surface of the treatment tip along a diagonal or in a grid distribution. In this way, not only whether the treatment tip is completely in contact with the skin can be determined, but also the contact uniformity can be detected, thereby avoiding that the treatment is performed while the treatment tip is not fully in contact with the skin due to misjudgment of the single-point pressure. In addition, the multi-sensor arrangement can ensure that effective contact is determined only when at least two sensors sense sufficient pressure simultaneously, thereby avoiding false triggering treatment when an angle forms at the contact position between the treatment tip and the skin or partial of the treatment tip contacts with the skin. Thus, the safety and effectiveness of the treatment can be significantly improved. In addition, the multi-sensor layout can also detect the movement state of the treatment tip on the skin, such as the sliding speed and the direction, to provide more comprehensive information for dynamically adjusting the treatment parameters, thereby ensuring that the treatment process is both efficient and accurate.

In summary, for the conventional treatment tip, the treatment is performed when the treatment tip contacts the skin, and the sliding speed during the user uses the treatment tip cannot match the corresponding operation parameters, resulting in poor treatment effect. Therefore, the present application proposes a method for controlling an operation parameter based on a pressure-sensitive sensor. The pressure-sensitive sensor is used in the treatment tip of a high-frequency output device. When the treatment tip contacts the skin, the pressure-sensitive sensor can generate a corresponding contact signal according to the contact mode between the treatment tip and the skin. Thus, the contact mode between the treatment tip and the skin can be determined by the contact signal, and the control module of the high-frequency output device can control the operation parameters of the treatment tip according to the contact mode. In this way, the corresponding work can be performed according to the contact mode between the treatment tip and the skin. When the treatment tip slides quickly or slowly, corresponding operation parameters (operation power and/or operation frequency) are set to ensure that the sliding speed matches the operation parameters. In addition, it also ensures that the treatment tip stops working when it is not needed, for example, when the treatment tip is in static contact with the skin. Thus, the treatment effect of the high-frequency output device or the treatment tip can be improved. In addition, since the present application only uses a pressure-sensitive sensor to detect the pressing force and the sliding speed, reducing the demand for the internal space of the treatment tip. In this way, compared with the treatment tip in the conventional technology, the present application reduces the internal space occupied by the treatment tip and improves the treatment effect of the treatment tip.

In an embodiment, the operation parameters include the operation power and the operation frequency. That is, the operation power and the operation frequency of the treatment tip are determined according to the contact condition between the treatment tip and the skin. In this way, the output frequency and the output power are matched according to the sliding speed, so that poor treatment effect or insignificant treatment effect caused by sliding too fast during use can be improved.

In an embodiment, the contact signal includes a value corresponding to the first signal and a value corresponding to the second signal, and the value corresponding to the first signal represents the sliding speed information after the treatment tip contacts the skin; the value corresponding to the second signal represents the pressing force information after the treatment tip contacts the skin.

It can be understood that the specific value of the first signal can be used to determine whether the treatment tip is moving quickly or slowly, or is completely stationary. The specific value of the second signal can be used to determine whether the treatment tip contacts the skin with appropriate pressure. By combining the value corresponding to the first signal and the value corresponding to the second signal, the control system can fully obtain the contact condition between the treatment tip and the skin, thereby more accurately adjusting the operation parameters of the treatment tip.

In this embodiment, step S20 may include: in response to that a value corresponding to the first signal is less than a first preset threshold, indicating that the treatment tip is in static contact with the skin, and outputting, by the high-frequency output device, a first parameter to the treatment tip.

It can be understood that static contact refers to a situation where there is almost no relative movement between the treatment tip and the skin. In this state, the treatment tip is in close contact with the skin, but there is no obvious sliding. The first parameter is an operation parameter set by the high-frequency output device according to the current contact mode. When it is detected that the treatment tip is in static contact with the skin, the high-frequency output device will output the first parameter to the treatment tip, so that the treatment tip works with the first parameter. In this embodiment, the operation power and the operation frequency corresponding to the first parameter can be 0 or slightly greater than 0. When it is 0, it can ensure that the treatment tip stops outputting when the treatment tip does not slide, avoiding excessive accumulation of ultrasonic energy in the same area and causing damage to the user. When the operation power and the operation frequency corresponding to the first parameter are greater than 0, single-point treatment can be achieved, so the first parameter can be an appropriate power and frequency, or it can correspond to the closed state of the treatment tip, which can be determined according to the actual application.

It should be noted that the first preset threshold and the second preset threshold mentioned below are both thresholds corresponding to the first signal, that is, representing the sliding speed change rate. The third preset threshold is the threshold corresponding to the second signal, that is, representing the contact strength.

In an embodiment, step S20 can also include: in response to that the value corresponding to the first signal is greater than the first preset threshold, indicating that the treatment tip is in dynamic contact with the skin, and outputting, by the high-frequency output device, a second parameter to the treatment tip.

It can be understood that dynamic contact refers to the situation where there is relative movement between the treatment tip and the skin. In this state, the treatment tip is sliding while the treatment tip is in contact with the skin. The second parameter is an operation parameter set by the high-frequency output device according to the current contact mode. When it is detected that the treatment tip is in dynamic contact with the skin, the high-frequency output device will output the second parameter to the treatment tip, so that the treatment tip works with the second parameter. In this embodiment, the power and the frequency corresponding to the second parameter are both greater than 0, that is, the treatment tip is in a treatment state. The value of the specific parameter depends on the sliding speed, which is not limited here. In this way, when the treatment tip is in dynamic contact with the skin, the system can control the treatment tip to perform working.

In this embodiment, step S20 can also include: in response to that a value corresponding to the first signal is greater than a first preset threshold and less than a second preset threshold, indicating that the treatment tip is in contact with the skin at a first sliding speed, outputting, by the high-frequency output device, a third parameter to the treatment tip.

It is understandable that in this case, the treatment tip contacts the skin at the first sliding speed, that is, there is certain sliding between the treatment tip and the skin, but the sliding speed is not very fast. In this case, the high-frequency output device will output the third parameter to the treatment tip to ensure that the treatment is effective and does not cause insufficient treatment due to the excessive sliding speed. The specific value of the third parameter depends on the actual application requirements to ensure the treatment effect.

Step S20 may also include: in response to that the value corresponding to the first signal is greater than the second preset threshold, indicating that the treatment tip is in contact with the skin at a second sliding speed, and outputting, by the high-frequency output device, a fourth parameter to the treatment tip.

It is understandable that in this case, the treatment tip contacts the skin at the second sliding speed, indicating that there is a faster sliding speed between the treatment tip and the skin. In this case, the high-frequency output device will output the fourth parameter to the treatment tip to ensure that the treatment tip can evenly apply treatment energy. The fourth parameter may include higher power and frequency. In this embodiment, the operation power and the operation frequency of the fourth parameter are greater than that of the third parameter, so as to ensure that nice treatment effect can be achieved even in the case of fast sliding. The first parameter, the second parameter, the third parameter and the fourth parameter can directly or indirectly indicate power or frequency.

The value relationship between the first sliding speed and the second sliding speed is: the second sliding speed is greater than the first sliding speed. In this way, the greater the speed, the higher the operation parameter outputted by the high-frequency output device.

The value relationship between the first preset threshold and the second preset threshold is: the second preset threshold is greater than the first preset threshold.

It should be noted that the first sliding speed and the second sliding speed can be converted from the change rate of voltage and/or current, and the first sliding speed mentioned here is not limited to a certain value. The first sliding speed and the second sliding speed can be interval values, and any value in the interval value of the second sliding speed is greater than any value in the interval value of the first sliding speed. That is, when the treatment tip slides at any speed in the interval value of the first sliding speed, the high-frequency output device can output the third parameter to the treatment tip. When the treatment tip slides at any speed in the interval value of the second speed, the high-frequency output device can output the fourth parameter to the treatment tip. In an embodiment, the first sliding speed and the second sliding speed can also be average values. That is, when the average speed of the treatment tip in a unit time is the first sliding speed, the high-frequency output device can output the third parameter to the treatment tip. When the average speed of the treatment tip in a unit time is the second sliding speed, the high-frequency output device can output the fourth parameter to the treatment tip.

In this embodiment, step S20 may further include: in response to that the value corresponding to the first signal is greater than a first preset threshold, and the value corresponding to the first signal changes continuously, an output power of the high-frequency output device changes continuously along with the change of the value corresponding to the first signal.

It can be understood that, in this case, the sliding speed of the treatment tip changes within a certain range, and the output power of the high-frequency output device will change continuously according to the change of the sliding speed. This means that when the sliding speed increases, the output power will also increase, and vice versa. This dynamic adjustment ensures that the treatment tip can maintain the best treatment effect at any sliding speed, while avoiding uneven treatment or over-treatment caused by changes in sliding speed. For example, when the speed increases from A to B, this is not a sudden change, but a continuous change from A to B. Correspondingly, the output power and the output frequency do not change suddenly from A' to B', but continuously change from A' to B', thus further ensuring the applicability of the treatment tip.

In this embodiment, step S20 may also include: in response to that a value corresponding to the second signal is less than a third preset threshold, indicating that the treatment tip does not effectively contact the skin, and outputting, by the high-frequency output device, a fifth parameter to the treatment tip.

It is understandable that, in this case, the pressing force between the treatment tip and the skin is not enough to perform the treatment. This may mean that although the treatment tip is in contact with the skin, the contact force is not enough to ensure effective treatment. In this case, the high-frequency output device will output the fifth parameter to the treatment tip, and the fifth parameter may correspond to the closed state of the treatment tip, thereby avoiding ineffective treatment or skin damage caused by improper treatment.

Step S20 may also include: in response to that the value corresponding to the second signal is greater than the third preset threshold, indicating that the treatment tip is in static contact with the skin, and outputting, by the high-frequency output device, a first parameter to the treatment tip.

It is understandable that, in this case, there is effective contact between the treatment tip and the skin. This means that the treatment tip contacts the skin with an appropriate pressing force and meets the requirement for performing treatment. In this case, the operation power and the operation frequency corresponding to the first parameter can be 0 or slightly greater than 0. When the operation power and the operation frequency corresponding to the first parameter are 0, it can ensure that the treatment tip stops outputting when it does not slide, to avoid hurting the user. When the operation power and the operation frequency corresponding to the first parameter are greater than 0, single-point treatment can be achieved, so the first parameter can be an appropriate power and an appropriate frequency, or the first parameter can correspond to the closed state of the treatment tip, which can be determined according to the actual application.

In addition, there can be the following settings. When the treatment tip is only in static contact with the skin, the treatment tip does not work to avoid hurting the skin. Only when the treatment tip is sliding, it starts to work to perform treatment.

In an embodiment, the step S20 can be included in the above embodiments, that is, the system has multiple judgments to control the treatment tip, so as to achieve more reasonable treatment.

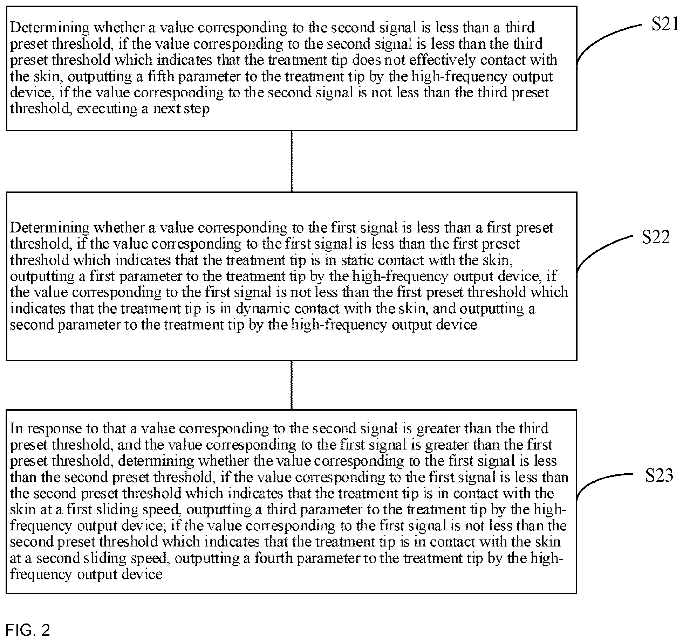

In an embodiment, as shown in FIG. 2, step S20 further includes steps S21 to S22.

S21, determining whether a value corresponding to the second signal is less than a third preset threshold, if the value corresponding to the second signal is less than the third preset threshold, indicating that the treatment tip does not effectively contact the skin, and outputting, by the high-frequency output device, a fifth parameter to the treatment tip, if the value corresponding to the second signal is not less than the third preset threshold, executing a next step.

S22, determining whether a value corresponding to the first signal is less than a first preset threshold, if the value corresponding to the first signal is less than the first preset threshold, indicating that the treatment tip is in static contact with the skin, and outputting, by the high-frequency output device, a first parameter to the treatment tip, if the value corresponding to the first signal is not less than the first preset threshold, indicating that the treatment tip is in dynamic contact with the skin, and outputting, by the high-frequency output device, a second parameter to the treatment tip.

In this way, the threshold ranges of the value corresponding to the first signal and the value corresponding to the second signal can be gradually judged from a small value to a large value, so as to control the operation of the treatment tip and the corresponding operation parameters. Through the multi-layer judgment logic, the high-frequency output device can more accurately identify the contact state between the treatment tip and the skin, and dynamically adjust the operation parameters according to the actual situation, thereby improving the accuracy and safety of the treatment.

It should be noted that, in the above embodiment, the operation parameters of the treatment tip can be controlled according to the value corresponding to the first signal and the value corresponding to the second signal in the contact signal simultaneously. By considering both the sliding speed and the pressing force, the control system can more comprehensively evaluate the contact state between the treatment tip and the skin. In this way, compared with the method using the value corresponding to one signal, the actual contact state between the treatment tip and the skin can be more accurately reflected, thereby achieving more accurate, safer and more effective treatment.

In addition, since the operation parameters of the treatment tip can be controlled according to the value corresponding to the first signal and the value corresponding to the second signal in the contact signal simultaneously, it can be determined whether the operation parameters need to be changed according to the pressing force of the treatment tip when sliding. In an embodiment, in the sliding treatment process, the pressing force is crucial to the treatment effect. Appropriate pressing force can help the treatment tip to better contact the skin surface, thereby improving the treatment effectiveness. Excessive pressure may cause skin damage, while the expected treatment effect may not be achieved due to too little pressure. Thus, the following examples can be used to illustrate the control method. First, when the treatment tip slides quickly and the pressure is moderate, the system can output the fourth parameter to the treatment tip to ensure that the treatment tip can still evenly apply treatment energy when it moves quickly. Second, when the treatment tip slides slowly and the pressure is too small, the system can output the second parameter or the third parameter to the treatment tip to reduce power and avoid ineffective treatment. When the treatment tip slides slowly and the pressure is too large, the system may output the fifth parameter to the treatment tip to reduce power and avoid local overheating or damage.

In an embodiment, as shown in FIG. 2, step S20 includes step S23.

S23, in response to that a value corresponding to the second signal is greater than the third preset threshold, and the value corresponding to the first signal is greater than the first preset threshold, determining whether the value corresponding to the first signal is less than the second preset threshold, if the value corresponding to the first signal is less than the second preset threshold, indicating that the treatment tip is in contact with the skin at a first sliding speed, and the high-frequency output device outputs a third parameter to the treatment tip, if the value corresponding to the first signal is not less than the second preset threshold, indicating that the treatment tip is in contact with the skin at a second sliding speed, and the high-frequency output device outputs a fourth parameter to the treatment tip.

Similarly, step S23 provides a more sophisticated control strategy by comprehensively considering the values corresponding to the two signals of the sliding speed and the pressing force, thereby ensuring the safety and effectiveness of the treatment process.

In this embodiment, when the treatment tip contacts the skin, the resistance value of the pressure-sensitive sensor changes, so that the corresponding operation voltage and/or operation current change accordingly. The first signal specifically includes the change rate of the operation voltage and/or operation current. The second signal specifically includes the amplitude of the operation voltage and/or operation current.

It can be understood that when the treatment tip contacts the skin, the resistance value of the pressure-sensitive sensor changes with the change of the contact pressure. Such a change will cause a corresponding change in the operation voltage and/or operation current in the circuit.

The first signal includes the change rate of the operation voltage and/or the operation current. The system can determine the sliding speed of the treatment tip after it contacts the target area based on the first signal. Specifically, when the treatment tip begins to contact the skin or moves on the skin, the resistance value of the pressure-sensitive sensor will change rapidly, causing the current or voltage in the circuit to change rapidly. This change rate is transmitted to the system in the form of the first signal to reflect the instantaneous dynamic or sliding speed of the treatment tip contacting the target area. For example, when the treatment tip slides quickly, the value corresponding to the first signal will be larger, indicating that the change rate of the operation voltage and/or the operation current is higher. Conversely, when the treatment tip is stationary or moves slowly, the value corresponding to the first signal will be smaller.

The second signal includes the amplitude of the operation voltage and/or the operation current. The system can judge the fitting degree between the treatment tip and the target area according to the first signal. Specifically, after the treatment tip is in stable contact with the skin, the resistance value of the pressure-sensitive sensor will suddenly change, and the corresponding operation voltage and/or operation current will also suddenly change, and will finally stabilize. The stabilized value is transmitted to the system in the form of the first signal to reflect the fitting degree (i.e., the pressing force) between the treatment tip and the target area. For example, if the final value (the value corresponding to the first signal) after stabilization exceeds the third preset threshold, it is determined to be a effective contact, otherwise it is determined to be an invalid contact.

In an embodiment, the first signal and the second signal are both converted into the ADC signals. The ADC signals can provide a more accurate, stable and easyto-process data format, which helps the high-frequency output device to realize intelligent and dynamic adjustment of the operation parameters of the treatment tip.

It can be understood that the first signal and the second signal are both corresponding voltage signals or current signals, and are both analog signals. The analog signals are converted into digital signals for analyzing and processing by a microprocessor or a control system.

It should be explained that the first preset threshold and the second preset threshold mentioned above can be set based on the fluctuation rate of the ADC signal to distinguish different types of sliding speeds. Specifically, the first preset threshold is used to distinguish the static contact with and the dynamic contact. The second preset threshold is used to further distinguish different sliding speed levels. The third preset threshold can be set based on the specific value of the ADC signal to determine whether there is effective contact between the treatment tip and the skin. The third preset threshold can be 200 or 500. The first preset threshold can be 200, and the second preset threshold is greater than 200.

In this embodiment, the first operation parameter represents that the treatment tip is in static contact with the skin, and the operation power and the operation frequency outputted by the high-frequency output device can be 0 or greater than 0. The second operation parameter represents that the treatment tip is in dynamic contact with the skin, and the operation power and the operation frequency outputted by the high-frequency output device can greater than 0. The third operation parameter represents that the treatment tip is in contact with the skin at the first sliding speed. The fourth operation parameter represents that the treatment tip is in contact with the skin at the second sliding speed. The second sliding speed is greater than the first sliding speed. The operation power and operation frequency corresponding to the fourth parameter are also greater than the operation power and operation frequency corresponding to the third parameter. The fifth operation parameter represents that the treatment tip is not in effective contact with the skin, and the treatment tip can be in the closed state.

In actual application, after the treatment tip contacts the skin, if the ADC value collection feedback data of the pressure-sensitive sensor remains basically unchanged or shows a small fluctuation range (less than the threshold of 200), it indicates that the treatment tip is in static contact state, and the treatment tip does not output energy. If the ADC value collection feedback data changes rapidly, it indicates that the treatment tip is sliding rapidly. If the ADC value collection feedback data changes slowly, it indicates that the treatment tip is sliding slowly.

For frequency matching based on the ADC value, after the treatment tip contacts the skin, if the ADC value collection feedback data of the pressure-sensitive sensor remains basically unchanged or shows a small fluctuation range (less than the threshold of 200), it indicates that the treatment tip is in static contact state, and the treatment tip does not output energy (corresponding to the first parameter as mentioned above). For the sliding state, the system matches the output frequency according to the change rate of the ADC value. When the ADC value changes rapidly (indicating that the treatment tip slides rapidly), the energy output frequency is set to 25 times per second (corresponding to the fourth parameter as mentioned above). When the ADC value changes slowly (indicating that the treatment tip slides slowly), the energy output frequency is set to 8 times per second (corresponding to the third parameter as mentioned above).

For power matching based on the ADC value, after the treatment tip contacts the skin, if the ADC value collection feedback data of the pressure-sensitive sensor remains basically unchanged or the fluctuation range is very small (less than the threshold of 200), it indicates that the treatment tip is in static contact state, and the treatment tip does not output energy. For the sliding state, the system matches the output power according to the change rate of the ADC value. When the ADC value changes quickly, it indicates that the treatment tip slides quickly, and the energy output power is set to 5 watts (corresponding to the fourth parameter as mentioned above). When the ADC value changes slowly, it indicates that the treatment tip slides slowly, and the energy output power is set to 2 watts (corresponding to the third parameter as mentioned above).

In a specific application scenario, the method disclosed in the present application is applied to a treatment tip, and the third preset threshold is set to 200. Then, if the ADC value corresponding to the second signal is greater than 200 after the treatment tip is attached to the target area, it is assumed that the treatment tip has been fitted in place. Subsequently, the value is detected every 100 milliseconds to see if the value has changed. If the value changes, the change rate is converted into the second signal, and the movement speed of the treatment tip is determined according to the ADC value corresponding to the second signal. The energy output of the treatment tip depends on the movement speed. For example, the shorter the time from one value to another, the faster the movement speed and the higher the synchronization speed of energy output. On the contrary, if the time is longer, the slower the movement speed and the lower the synchronization speed of energy output.

The present application also proposes a treatment tip, which includes: a pressure-sensitive sensor; a memory for storing a computer program; a processor for executing the computer program. The computer program is configured to implement any of the above method for controlling an operation parameters based on the pressure-sensitive sensor.

The treatment tip includes a memory and a processor, which are used to store and implement a method for controlling an operation parameter based on a pressure-sensitive sensor. The specific implementation of the method for controlling an operation parameter based on the pressure-sensitive sensor refers to the above embodiments. Since the memory and the processor adopt all the technical solutions of all the above embodiments, they at least have all the beneficial effects brought by the technical solutions of the above embodiments, which will not be repeated here.

The present application also proposes a high-frequency output device, which includes a treatment tip and a pressure-sensitive sensor. The pressure-sensitive sensor is arranged at the treatment tip, and the treatment tip is used to contact the skin.

In an embodiment, the high-frequency output device also includes: a feedback module, a processing module and a control module. The feedback module is used to receive and transmit a contact signal outputted by a pressure-sensitive sensor. The processing module is electrically connected to the feedback module, and is used to receive and process the contact signal to generate a corresponding processing signal. The control module is electrically connected to the processing module and the treatment tip, and is used to receive the processing signal and control the operation parameters outputted by the high-frequency output device according to the processing signal and the method for controlling an operation parameter based on the pressure-sensitive sensor as mentioned in any one of the above embodiments.

The above are only some embodiments of the present application, and do not limit the scope of the present application thereto. Under the concept of this application, any equivalent structural transformation made according to the description and drawings of the present application, or direct/indirect application in other related technical fields shall fall within the claimed scope of the present application.

## Claims

1. A method for controlling a high-frequency output device based on a pressure-sensitive sensor, **characterized in that**, the high-frequency output device comprises a treatment tip configured to contact skin and transmit high-frequency energy to the skin, the pressure-sensitive sensor is provided at the treatment tip, and the method comprises:
step S10, obtaining a contact signal collected by the pressure-sensitive sensor, wherein the contact signal is generated when the treatment tip contacts the skin; and
step S20, determining a contact condition between the treatment tip and the skin according to the contact signal, to control the high-frequency output device to output an operation power and/or an operation frequency with a corresponding value to the treatment tip.

2. The method according to claim 1, wherein the contact signal comprises a first signal and a second signal, the first signal represents sliding speed information of the treatment tip contacting with the skin, and the second signal represents pressing force information of the treatment tip contacting with the skin.

3. The method according to claim 2, wherein step S20, determining the contact condition between the treatment tip and the skin according to the contact signal, to control the high-frequency output device to output the operation power and/or the operation frequency with the corresponding value to the treatment tip, further comprises:
in response to that a value corresponding to the first signal is less than a first preset threshold which indicates that the treatment tip is in static contact with the skin, outputting a first parameter to the treatment tip by the high-frequency output device;
in response to that the value corresponding to the first signal is greater than a first preset threshold which indicates that the treatment tip is in dynamic contact with the skin, outputting a second parameter to the treatment tip by the high-frequency output device.

4. The method according to claim 2, wherein step S20, determining the contact condition between the treatment tip and the skin according to the contact signal, to control the high-frequency output device to output the operation power and/or the operation frequency with the corresponding value to the treatment tip, further comprises:
in response to that a value corresponding to the first signal is greater than a first preset threshold and less than a second preset threshold which indicates that the treatment tip is in contact with the skin at a first sliding speed, outputting a third parameter to the treatment tip by the high-frequency output device;
in response to that the value corresponding to the first signal is greater than a second preset threshold which indicates that the treatment tip is in contact with the skin at a second sliding speed, outputting a fourth parameter to the treatment tip by the high-frequency output device.

5. The method according to claim 2, wherein step S20, determining the contact condition between the treatment tip and the skin according to the contact signal, to control the high-frequency output device to output the operation power and/or the operation frequency with the corresponding value to the treatment tip, further comprises:
in response to that a value corresponding to the first signal is greater than a first preset threshold, and the value corresponding to the first signal changes continuously, an output power of the high-frequency output device changes continuously along with a change of the value corresponding to the first signal.

6. The method according to claim 2, wherein step S20, determining the contact condition between the treatment tip and the skin according to the contact signal, to control the high-frequency output device to output the operation power and/or the operation frequency with the corresponding value to the treatment tip, further comprises:
in response to that a value corresponding to the second signal is less than a third preset threshold which indicates that the treatment tip does not effectively contact with the skin, outputting a fifth parameter to the treatment tip by the high-frequency output device;
in response to that the value corresponding to the second signal is greater than a third preset threshold which indicates that the treatment tip is in static contact with the skin, outputting a first parameter to the treatment tip by the high-frequency output device.

7. The method according to claim 2, wherein step S20, determining the contact condition between the treatment tip and the skin according to the contact signal, to control the high-frequency output device to output the operation power and/or the operation frequency with the corresponding value to the treatment tip, further comprises:
step S21, determining whether a value corresponding to the second signal is less than a third preset threshold, if the value corresponding to the second signal is less than the third preset threshold which indicates that the treatment tip does not effectively contact with the skin, outputting a fifth parameter to the treatment tip by the high-frequency output device, if the value corresponding to the second signal is not less than the third preset threshold, executing a next step; and
step S22, determining whether a value corresponding to the first signal is less than a first preset threshold, if the value corresponding to the first signal is less than the first preset threshold which indicates that the treatment tip is in static contact with the skin, outputting a first parameter to the treatment tip by the high-frequency output device, if the value corresponding to the first signal is not less than the first preset threshold which indicates that the treatment tip is in dynamic contact with the skin, and outputting a second parameter to the treatment tip by the high-frequency output device.

8. The method according to claim 7, wherein after step S22, determining whether a value corresponding to the first signal is less than a first preset threshold, if the value corresponding to the first signal is less than the first preset threshold which indicates that the treatment tip is in static contact with the skin, outputting a first parameter to the treatment tip by the high-frequency output device, if the value corresponding to the first signal is not less than the first preset threshold which indicates that the treatment tip is in dynamic contact with the skin, and outputting a second parameter to the treatment tip by the high-frequency output device, the method further comprises:
step S23, in response to that the value corresponding to the second signal is greater than the third preset threshold, and the value corresponding to the first signal is greater than the first preset threshold, determining whether the value corresponding to the first signal is less than the second preset threshold, if the value corresponding to the first signal is less than the second preset threshold which indicates that the treatment tip is in contact with the skin at a first sliding speed, outputting a third parameter to the treatment tip by the high-frequency output device, if the value corresponding to the first signal is not less than the second preset threshold which indicates that the treatment tip is in contact with the skin at a second sliding speed, outputting a fourth parameter to the treatment tip by the high-frequency output device;
wherein the second preset threshold is greater than the first preset threshold, and the second sliding speed is greater than the first sliding speed.

9. The method according to any one of claims 2 to 8, wherein,
the first signal indicates a change rate of an operation voltage and/or an operation current; and
the second signal indicates an amplitude of the operation voltage and/or the operation current.

10. A treatment tip, **characterized by** comprising:
a pressure-sensitive sensor;
a memory for storing a computer program; and
a processor for executing the computer program,
wherein the computer program is configured to implement the method for controlling a high-frequency output device based on a pressure-sensitive sensor according to any one of claims 1 to 9.

11. A high-frequency output device, **characterized by** comprising:
an output module;
a feedback module;
a control module; and
the treatment tip according to claim 10;
wherein the feedback module is configured to receive and transmit a contact signal outputted by the pressure-sensitive sensor of the treatment tip, the control module is electrically connected to the feedback module and the treatment tip, and the control module is configured to receive a contact signal to control the output module to output an operation power and/or an operation frequency with a corresponding value to the treatment tip.
